# EUROPÄISCHE PATENTSCHRIFT

(11) **EP 1 809 208 B1**
(45) Veröffentlichungstag und Bekanntmachung des Hinweises auf die Patenterteilung: **01.07.2015**
(21) Anmeldenummer: 05808158.9
(22) Anmeldetag: 09.11.2005
(51) Int. Cl.: A61F 2/34, B23G 1/00, G05B 19/18, F16B 25/10, F16B 33/02, F16C 11/06

(54) **EINSCHRAUBKÖRPER MIT SICH ÄNDERNDEM GEWINDEPROFIL UND VERFAHREN ZU DESSEN HERSTELLUNG**
SCREW-IN MEMBER COMPRISING A CHANGING THREAD PROFILE, AND METHOD FOR THE PRODUCTION THEREOF
CORPS A VISSER PRESENTANT UN PROFIL FILETE VARIABLE ET SON PROCEDE DE PRODUCTION

(30) Priorität: 09.11.2004 DE 102004053944; 30.11.2004 DE 102004057709; 30.05.2005 DE 102005024699
(43) Veröffentlichungstag der Anmeldung: 25.07.2007
(73) Patentinhaber: Hörmansdörfer, Gerd, 31303 Burgdorf-Beinhorn (DE)
(72) Erfinder: Hörmansdörfer, Gerd, 31303 Burgdorf-Beinhorn (DE)
(74) Vertreter: Siekmann, Gunnar
(86) Internationale Anmeldenummer: PCT/DE2005/002013
(87) Internationale Veröffentlichungsnummer: WO 2006/050707

(56) Entgegenhaltungen:
- EP-A- 0 480 551
- WO-A-00/75737
- WO-A-95/18586
- WO-A-97/39702
- US-A- 5 120 171
- US-A- 5 628 630
- US-A1- 2004 121 289

## Beschreibung

Die Erfindung betrifft einen Einschraubkörper mit einer mindestens in einem Teilbereich nicht-linearen insbesondere geknickten oder gekrümmten Mantelfläche und einem mindestens in diesem Teilbereich liegenden Gewinde mit sich änderndem, aus Gewindezahn und Gewinderille gebildetem Gewindeprofil, wobei die in Einschraubrichtung liegende Flanke des Gewindezahns einen konstanten Winkel besitzt. Weiterhin betrifft die Erfindung ein Verfahren zur spanenden Herstellung von Gewinden mit sich mindestens in einem Teilbereich veränderndem Gewindeprofil auf einer CNC-Maschine, vorzugsweise einer CNC-Drehmaschine, wobei das Gewinde mindestens in diesem Teilbereich mit mindestens zwei Herstellungsgängen bzw. - zyklen bearbeitet wird. Gewinde sind als konstruktive Elemente des allgemeinen Maschinenbaus weit verbreitet. Gewöhnlich sind Gewinde zylinderförmig gestaltet. Daneben sind auch konische Gewinde z.B. für Ölfeldrohre verbreitet. Eine große Anzahl verschiedener Gewindeprofile ist bekannt und in Normen festgelegt. Üblicherweise ist das Gewindeprofil an einem Werkstück unveränderlich, das heißt, dass das Gewindeprofil am Gewindeanfang identisch mit dem am Gewindeende ist. Es sind jedoch Ausnahmen vorstellbar, bei denen eine zumindest in einem Teilbereich des Gewindes sich fließend ändernde Formgestalt des aus Gewinderille und Gewindezahn gebildeten Gewindeprofils von Vorteil sein könnte, z.B. um das Einführen eines Schraubengewindes in ein Mutterngewinde zu erleichtern.

Spezielle geometrische Verhältnisse in Bezug auf das Gewinde bestehen jedoch vor allem bei Gewinden auf gekrümmten Flächen, wie sie insbesondere bei einschraubbaren künstlichen Hüftgelenkpfannen vorkommen. Hier sind hinsichtlich der Mantelform des Schalenkörpers z.B. hypo-, hemi-, oder hypersphärische, konisch-sphärische, parabolische, toroidische, elliptische und ähnliche Geometrien bekannt. Bei spanenden Herstellungsverfahren für die Herstellung derartiger Schraubpfannen ergeben sich teilweise zwangsläufig sich fließend verändernde Verzerrungen des Gewindeprofils, welche in den meisten Fällen weder beabsichtigt noch erwünscht sind. Insbesondere bei der Benutzung von Gewindezähnen mit unsymmetrischen Teilflankenwinkeln (den jeweiligen Seitenwinkeln des Gewindezahns) ergibt sich das Phänomen, dass je nach Kipprichtung des resultierenden Gewindezahns die Zahnhöhe vom Pfannenäquator in Richtung zum Pfannenpol hin fließend zu- bzw. abnimmt, woraus sich dann am polnahen Gewindeanfang entweder viel zu große oder nahezu verkümmerte Gewindezähne ergeben. Im ersten Fall führen die extrem großen Gewindezähne dazu, dass zum Einschrauben der Pfanne sehr hohe Kräfte erforderlich werden, bzw. das Implantat nicht bis zum vollständigen Knochenkontakt eingeschraubt werden kann. Im zweiten Fall wird lediglich eine sehr dürftige Primärfixation zu erreichen sein. In beiden Fällen besteht die Gefahr des Auslockerns des Implantats, welches als Konsequenz eine nochmalige Operation des Patienten bedeuten würde.

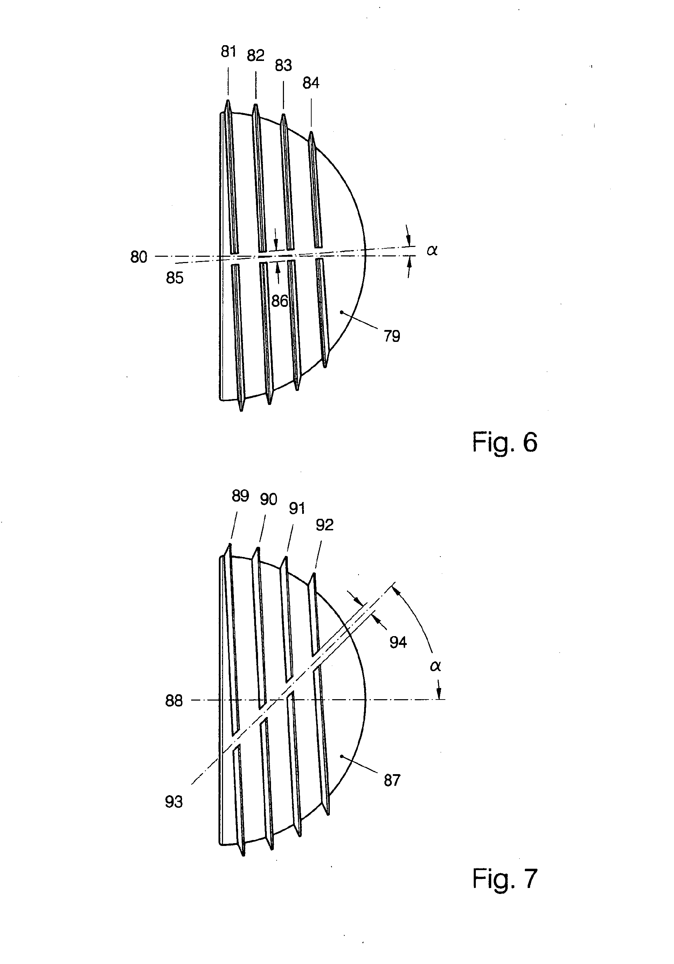

Aus der französischen Patentanmeldung 2 548 012 ist eine Schraubpfanne bekannt, deren äußere Form im Anmeldetext als zylindrisch-sphärisch beschrieben ist. Sie ist mit einem eingängigen Gewinde ausgestattet, welches sich über die gesamte äußere Länge erstreckt und dessen Gewindeprofil sich vom Pfannenäquator zum Pfannenpol hin fließend ändert. Diese Profiländerung kommt in einer Abmagerung der Zahnhöhe und einer zunehmenden Ungleichheit der Zahnflankenlängen in Richtung zum Pfannenpol hin zum Ausdruck. Das Gewinde ist als herkömmliches ISO-Gewinde mit einem eingeschlossenen Flankenwinkel von 60° gefertigt. Davon wird in den Zeichnungsfiguren sozusagen das Ergebnis des letzten Schnitts mit einem üblichen Werkzeug (z.B. Gewindeschneidplatte) gezeigt. Zwecks Erzielung einer in Bezug auf die Gewindezahnspitzen konstanten Gewindesteigung wird vorgeschlagen, während der Herstellung auf einer CNC-Maschine das Werkzeug auf einer Bahn mit einer sich bei jeder Umdrehung ändernden Steigung zu verfahren. Der geometrische Hintergrund zur Ermittlung der jeweiligen Steigung ist in Fig.6 der genannten Anmeldung zeichnerisch dargestellt. Auf den ersten Blick erscheint die angegebene Vorgehensweise plausibel und ohne weiteres umsetzbar.

Bei näherer Analyse fällt zunächst auf, dass zwar die Gewindespitzen und der jeweilige Gewindegrund in der zweidimensionalen Darstellung auf Kreisbögen liegen, diese jedoch mit ihrem Drehpunkt nicht mit der Pfannenachse übereinfallen. Daher ist weder für den den Gewindegrund noch für den die Zahnspitzen einhüllenden geometrischen Mantel eine Kugelform realisiert. Eine präzise zeichnerische Darstellung mit einem CAD-System beweist dann, dass die ins Auge gefaßte Herstellungsmethode keinesfalls in der Lage ist, das dargestellte Gewindezahnprofil zu realisieren. Mit zunehmendem Abtauchen der Gewinderille zum Pfannenpol hin treten nämlich gravierende geometrische Effekte auf, welche sich in extremen Fluktuationen der Gewindezahnhöhe auswirken. In der von Hand gefertigten Zeichnungsfigur der Anmeldeschrift sind diese drastischen Fehler nicht sichtbar, weil sie durch eine Summierung einer größeren Zahl zeichnerischer Ungenauigkeiten ausgeglichen werden.

Tatsächlich ist es jedoch nicht möglich, mit dem unveränderlichen Gewindeprofil eines das Gewinde schneidenden Werkzeugs bei einem einzügigen Endschnitt mehreren voneinander unabhängigen Gesetzmäßigkeiten zu folgen.

Daneben ist das vorgeschlagene Gewinde mit ISO-Profil für den vorgesehenen Zweck sowieso ungeeignet, weil es einerseits dem Festigkeitsverhältnis zwischen dem Knochen und dem Implantatwerkstoff nicht Rechnung trägt und andererseits sehr hohe Einschraubkräfte beim Implantieren erfordert. Selbst bei Abänderung des Gewindeprofils, z.B. durch Verwendung eines Drehmeißels mit trapezförmigem Profil und einer damit einhergehenden Verschmälerung der Gewindezähne, wäre bei einer einzügigen Herstellungsweise der Gewindegrund durch Treppenstufen repräsentiert. Damit wären nicht nur Klemmeffekte beim Einschrauben verbunden, sondern auch eine unerwünschte Spaltenbildung im Kontaktbereich zur knöchernen Lagerfläche. Diese Problematik besteht im Prinzip für viele Arten von Einschraubkörpern, welche einerseits über eine mindestens in einem Teilbereich gekrümmte Mantelfläche verfügen, und andererseits für das Einschrauben in ein Material mit einer gegenüber derjenigen des Einschraubkörpers deutlich reduzierten Festigkeit verfügen.

Der oben geschilderte Sachverhalt ist Gegenstand der Patentschrift WO 95/18586 A und daher allgemein bekannt. In dieser Schrift wird vorgeschlagen, zur Erzielung eines bestimmten Gewindeprofilverlaufs das Gewinde mit unterschiedlichen Steigungen zu bearbeiten. Dieses Verfahren wird bereits im Bereich der Medizintechnik für die Herstellung von Knochenschrauben und künstlichen Hüftgelenkpfannen mit Erfolg angewandt. Dabei werden die Schutzrechte der zugehörigen Patentfamilie von den jeweiligen Herstellern in Bezug auf die jeweilige Produktgruppe exklusiv genutzt. Allerdings ist die mit dem Verfahren zur Verfügung stehende Bandbreite der Gestaltungsmöglichkeiten auf die Verwendung unterschiedlicher Gewindesteigungen beschränkt. Eine irgendwie geartete Beeinflussung des Kippwinkels des Gewindezahns ist nicht möglich.

Aus der Offenlegungsschrift DE 33 25 448 ist nun eine Hüftgelenkpfanne mit selbstschneidendem Gewinde und bombierter Kontur des Schalenmantels bekannt, welche ein im Prinzip konstantes Profil des Gewindezahns mit einer jeweils senkrechten Positionierung auf einer auf den Schalenmantel angelegten Tangente besitzt. Entsprechend unterliegen beide Flanken des Gewindezahns einer in Richtung zum Pfannenpol synchron zunehmenden Kippung. Dabei ragen die Zahnspitzen auf der Winkelhalbierenden mit einer jeweils identischen Höhe aus dem Schalenmantel heraus. Dieses Gewinde wird mit Hilfe einer Mehrachsenfräsmaschine produziert.

Eine sorgfältige Überprüfung der bei der genannten Schraubpfanne bestehenden Einschraubsituation mittels einer 2D-Simulation auf einem Rechner zeigt allerdings, dass eine derartige Gewindegestaltung nicht optimal funktionieren kann. Der wesentliche Mangel besteht im Auftreten eines mindestens einseitigen Spalts zwischen Gewindezahn und der knöchernen Lagerfläche, weil während des Einschraubprozesses eine Furche erzeugt wird, welche breiter ist als das Gewindezahnprofil selbst. Die vorliegende Erfindung offenbart einen Einschraubkörper gemäß Anspruch 1 sowie ein Verfahren zur spanenden Herstellung von Gewinden gemäß Anspruch 9. Weitere Ausführungsformen sind in den davon abhängigen Ansprüchen definiert.

Es bestand daher die Aufgabe zur Schaffung von spanend auf einer CNC-Drehmaschine herstellbaren, alternativen Einschraubkörpern (z.B. in Gestalt von Knochenschrauben oder Hüftgelenkpfannen) mit einem mindestens in einem Teilbereich auf einer gekrümmten oder abgewinkelten Mantelfläche liegenden Gewinde mit unsymmetrischen Flanken des Gewindezahns und einer entlang der Gewindeerstreckung anpaßbaren Gewindezahnhöhe, sowie eines Verfahrens zur Herstellung solch eines speziellen Gewindes für diese und andere Anwendungen auf einer CNC-Maschine, vorzugsweise auf einer CNC-Drehmaschine.

Bei einem Einschraubkörper mit einer mindestens in einem Teilbereich nicht-linearer, insbesondere geknickter oder gekrümmter Mantelfläche und einem mindestens in diesem Teilbereich liegenden Gewinde mit sich änderndem, aus Gewindezahn und Gewinderille gebildeten Gewindeprofil, wobei die in Einschraubrichtung liegende Flanke des Gewindezahns einen konstanten Winkel besitzt, ist es erfindungswesentlich, dass die andere Flanke des Gewindezahns im genannten Teilbereich einer Winkeländerung unterliegt.

In einer bevorzugten Ausgestaltung der Erfindung ist die in Einschraubrichtung liegende Flanke des Gewindezahns subvertikal, vorzugsweise senkrecht, zur Achse des Einschraubkörpers ausgerichtet. Weiterhin ist es günstig, dass die Winkeländerung der anderen Flanke kontinuierlich, insbesondere im wesentlichen fließend, erfolgt. Die Gewindezähne sind günstigerweise derart ausgebildet, dass dem Verlauf der Gewindezähne eine jeweils aus mindestens einer Rampen- und einer Stufenfunktion gebildete geometrische Funktion überlagert ist. Zudem besteht eine bevorzugte Ausgestaltung darin, dass die Höhe des Gewindezahnprofils relativ zur Mantelfläche des Einschraubkörpers im hinter einer jeweiligen Schneidkante liegenden Bereich abnimmt. Bevorzugt ist die Schneidkante jedes Zahns im Vergleich zur Schneidkante des vorhergehenden Zahns äquatorseitig vorstehend ausgebildet. Durch seitlichen Versatz wird ein gutes Schneidverhalten erreicht. Der Einschraubkörper ist bevorzugt als Implantat und insbesondere bevorzugt als künstliche Hüftgelenkpfanne realisiert.

Günstig ist weiterhin, dass die Gewinderille eines auf einer mindestens partiell gekrümmten oder abgewinkelten Mantelfläche eines Einschraubkörpers liegenden Gewindes in mindestens zwei streifenförmige Teilflächen aufgegliedert wird, von denen die erste durch die zum Pfannenpol zeigende Flanke des Gewindezahns und die zweite durch die zum Pfannenäquator zeigende Flanke des Gewindezahns gebildet ist, und die erste Flanke entlang der Gewindeerstreckung in ihrem Winkel unveränderlich ist, während die zweite Flanke mindestens in einem Teilbereich entlang der Erstreckung des Gewindes eine Winkeländerung aufweist, wodurch die Höhe und/oder der Kippwinkel des Gewindezahns an die konstruktive Vorgabe angepaßt ist. Mit der Erfindung wird ferner ein Verfahren zur Herstellung solch eines speziellen Gewindes auf einer CNC-Maschine, z.B. einer CNC-Drehmaschine, vorzugsweise mit Werkzeugantrieb, zur Verfügung gestellt.

Das erfindungsgemäße Verfahren zur spanenden Herstellung von Gewinden mit sich mindestens in einem Teilbereich verändernden Gewindeprofil auf einer CNC-Maschine, vorzugsweise einer CNC-Drehmaschine, wobei das Gewinde mindestens in diesem Teilbereich mit mindestens zwei Herstellungsgängen bzw. - zyklen bearbeitet wird, zeichnet sich dadurch aus, dass bei einem dieser Herstellungsgänge bzw. - zyklen die die Gewindezahnflanke schneidende Kante des Drehmeißels einen festen Winkel einnimmt, und bei dem anderen Herstellungsgang bzw. - zyklus die die andere Gewindezahnflanke schneidende Kante des Drehmeißels in ihrem Winkel während des Durchlaufs durch die Gewinderille geändert wird.

In einer weiteren Ausgestaltung des erfindungsgemäßen Verfahrens betrifft der eine Bearbeitungsgang bzw. - zyklus im wesentlichen die eine Flanke des Gewindezahns sowie einen Teil des benachbarten Gewindegrundes und der andere Bearbeitungsgang bzw. - zyklus betrifft im wesentlichen die andere Flanke des Gewindezahns sowie einen Teil des dieser anderen Flanke benachbarten Gewindegrundes. Besonders günstig ist es, dass das Gewinde mit drei verschiedenen Bearbeitungsgängen bzw. - zyklen bearbeitet wird, wovon mit dem dritten Bearbeitungsgang bzw. - zyklus im wesentlichen die Mitte der Gewinderille bearbeitet und dabei eine Winkeleinstellung für den Drehmeißel benutzt wird, welche zwischen den für die beiden anderen Bearbeitungsgänge bzw. - zyklen benutzten Winkeleinstellungen liegt. Besonders bevorzugt wird der dritte Bearbeitungsgang zuerst ausgeführt. Verfahrenstechnisch ist es auch günstig, dass die Winkeländerung des die andere Gewindezahnflanke schneidenden Drehmeißels während des Durchlaufs durch die Gewinderille kontinuierlich, insbesondere im wesentlichen fließend, erfolgt. Ein anderer Aspekt besteht darin, dass die Winkeländerung des Drehmeißels mittels Drehung der B-Achse der CNC-Maschine, vorzugsweise einer CNC-Drehmaschine, realisiert ist. Eine besondere Ausgestaltung des Verfahrens liegt darin, dass zuerst die vom Schneidenradiusmittelpunkt des Drehmeißels während des Abfahrens der in Gestalt des Gewinderillengrundes zu realisierenden Mantelfläche des herzustellenden Einschraubkörpers zu beschreibende Bahn berechnet wird, diese Bahn mit einem jeweiligen Offset für X und Z in Bezug auf den Abstand des Schneidenradiusmittelpunktes des Drehmeißels vom Rotationsmittelpunkt der B-Achse des Kreuzschlittens und für B in Bezug auf die Winkeldifferenz zwischen der Schneide des Drehmeißels und der Nullstellung der B-Achse umgerechnet und im CNC-Programm angewendet wird, und in der Werkstückbeschreibung eine Äquidistante verwendet wird, welche in ihrem Betrag dem Schneideradius des Drehmeißels entspricht. Ein weiterer Aspekt des Verfahrens liegt darin, dass der Relativbewegung zwischen Werkstück und Werkzeug eine mit der Zahnfolge synchronisierte Rampen- und Stufenfunktion überlagert ist. Günstigerweise erfolgt diese Zerspanung frästechnisch.

Dabei wird vorgeschlagen, die Gewinderille entlang der durch den Mantel des Einschraubkörpers gebildeten Kontur (dies ist im Prinzip der Grund der Gewinderille) in dem zur Anpassung des Gewindeprofils vorgesehenen Bereich in mindestens zwei Herstellungsgängen zu bearbeiten, wobei ein Bearbeitungsgang im wesentlichen die zum Pfannenpol zeigende Flanke und ein weiterer Bearbeitungsgang im wesentlichen die zum Pfannenäquator zeigende Flanke des Gewindezahns erzeugt, und dabei für die zum Pfannenäquator zeigende Flanke der Teilflankenwinkel mindestens in einem Teilbereich der Gewindeerstreckung geändert wird.

Für die praktische Ausführung des Verfahrens, z.B. auf einer CNC-Drehmaschine, können wahlweise entweder dasselbe Werkzeug oder zwei bzw. mehrere Werkzeuge Verwendung finden. Soll nur ein Werkzeug eingesetzt werden, so muss seine schneidende Kante mit ihrem Winkel jeweils derart positioniert sein, dass dieser dem Teilflankenwinkel des Gewindezahns entspricht. Üblicherweise kommt für derartige Bearbeitungen als Werkzeug eine Wendeplatte aus Vollhartmetall zum Einsatz, welche aufgrund ihrer genormten Geometrie einen bestimmten Eckenwinkel (z.B. 35°, 55° oder 75°) einschließt. Auf einem neutralen Halter würde die Wendeplatte dann mit ihrer Winkelhalbierenden senkrecht zur Achse des Gewindes stehen. Relativ zu der Winkelhalbierenden würde der Winkel der schneidenden Kante somit die Hälfte des Ekkenwinkels betragen. Bekannte Methoden zur festen Anpassung des Schneidkantenwinkels bestehen darin, den Halter entweder entsprechend abzufräsen, Distanzstücke unterzulegen, oder ihn schräg einzuspannen. Mit der Erfindung wird eine wesentlich elegantere Methode anwendbar, wenn z.B. eine CNC-Drehmaschine mit einer steuerbaren B-Achse des Kreuzschlittens zur Verfügung steht. Der Halter mit der Wendeplatte kann dann mittels einer entsprechenden Programmierung der B-Achse in die gewünschte Winkelstellung gebracht werden. Diese als statisch einzuordnende Winkelstellung kann z.B. nach jedem einzelnen Durchlauf beim Gewindeschneiden auf einen anderen Winkelbetrag gesetzt werden. Um jedoch die der Erfindung gemäße dynamische Winkelverschiebung zu realisieren, ist es erforderlich, den B-Winkel während des Durchlaufs des Werkzeugs durch die Gewinderille zu ändern. Zum Beispiel könnte einer der Teilflankenwinkel eines Gewindezahns am Anfang des Gewindes einen Betrag von 20° und am Ende einen solchen von 12° besitzen. Durch das wahlweise bezüglich der rechten und/oder linken Gewindezahnflanke und kontinuierlich bis sprunghaft programmierbare Schwenken dieses Winkels wird die Möglichkeit eröffnet, sowohl die Höhe als auch den Kippwinkel des Gewindezahns in nahezu beliebiger Weise, also auch fließend, an die konstruktiven Vorgaben anzupassen.

Die Erfindung erlaubt hier die Wahl, entweder ein Werkzeug zu verwenden, welches in seinem Profil dem Profil der Gewinderille an ihrer engsten Stelle entspricht, oder ein Werkzeug, welches generell schmaler als die Gewinderille ist. Im zweiten Fall kann für den Gewindeschnitt neben der Benutzung mindestens eines sich ändernden Teilflankenwinkels für die Bearbeitungsgänge zusätzlich ein entsprechend angepaßter Offset-Wert in der Steigungsachse einbezogen werden, um den Unterschied zwischen Werkzeug- und Gewinderillenbreite auszugleichen. Diese Vorgehensweise ist besonders vorteilhaft, weil damit z.B. eine kleinere Spitzenverrundung des Bearbeitungswerkzeugs ermöglicht wird. Dadurch kann bei Anwendung eines jeweils kleinen relativen Versatzes des Werkzeugs nach jedem Durchgang eines Gewindeschneidzyklusses der Gewindegrund feiner aufgelöst und der gekrümmten oder geknickten Mantelfläche besser angepaßt werden.

Mit der Erfindung wird ferner die Wahlmöglichkeit angeboten, die Gewindebearbeitung mit mehr als zwei Herstellungsgängen, bzw. mit zwei oder mehreren Werkzeugen durchzuführen. Werden z.B. drei Herstellungsgänge angewandt, so wird empfohlen, bei einem der Herstellungsgänge im wesentlichen die eine Seite der Gewinderille, bei einem weiteren im wesentlichen die andere Seite der Gewinderille, und bei einem dritten Herstellungsgang im wesentlichen den Gewindegrund zu bearbeiten. Dabei wird für das die Seiten der Gewinderille und damit die jeweiligen Flanken der Gewindezähne schneidende Werkzeug eine Positionierung gewählt, welche den jeweiligen Teilflankenwinkel ergibt, während vorgeschlagen wird, für das im wesentlichen die Mitte des Gewindegrundes schneidende Werkzeug eine Positionierung zu benutzen, welche mit ihrem Winkel zwischen diesen beiden Positionierungen liegt. Mit steigender Zahl der Bearbeitungsgänge bzw. Werkzeuge ist es so bei entsprechend kleiner Verrundung der jeweiligen Werkzeugspitze möglich, eine relativ kleine Verrundung des Gewindezahnfußes und eine noch bessere Anpassung des Gewindegrundes an die angestrebte Kontur der Mantelfläche eines derartigen Einschraubkörpers zu erzielen.

Das erfindungsgemäße Verfahren ist für die Anwendung auf einer per Rechner gesteuerten Maschine vorgesehen, insbesondere einer CNC-Drehmaschine. Beim Gewindedrehen auf derartigen Maschinen ist es üblich, die Gewinderille in mehreren Durchgängen mittels eines Drehmeißels zu bearbeiten. Der Herstellungsgang wird dann als Gewindeschneidzyklus bezeichnet. Bei einem einzigen Durchgang wird nur wenig Material abgetragen, dafür kann jedoch mit hohen Vorschüben gearbeitet werden. Demgegenüber kann ein Herstellungsgang beim Gewindefräsen aus einem einzigen Durchlauf bestehen, wofür sich jedoch aufgrund des erforderlichen kleinen Vorschubs in der Regel ein im Vergleich zum Drehen deutlich höherer Zeitaufwand ergibt. Für das Fertigen des erfindungsgemäßen Gewindes sind auf jeden Fall nur solche CNC-Maschinen bzw. CNC-Drehmaschinen geeignet, welche über eine Schwenkachse für das Werkzeug in der aus Längsachse (Z) und radialer Achse (X) des Werkstücks gebildeten Ebene verfügen. Im entsprechenden CNC-Programm muss die von dem jeweiligen Werkzeug bzw. für den jeweiligen Zyklus relativ zum Werkstück zurückzulegende Bahn abgelegt sein. Zur Synchronisation der Herstellungsgänge bzw. Werkzeuge ist es erforderlich, die unterschiedlichen Startpunkte für die einzelnen Herstellungsgänge bzw. Schneidzyklen genau zu berechnen und einzugeben. In den Programmsätzen müssen neben den üblichen Parametern die den Winkeln gemäßen Werte für die oben genannte Schwenkachse (B) zusätzlich abgelegt sein.

Bei der Herstellung eines der Erfindung gemäßen Einschraubkörpers auf einer CNC-Drehmaschine ist eine bestimmte Vorgehensweise angesagt. Dazu wird zunächst die Gewinderille grob mit einem geeigneten Stechmeißel ausgeräumt. Danach kann ein erster kürzerer Gewindezyklus ablaufen, bei welchem ein Schlichtmeißel zur Tiefe hin in der X-Achse so lange zugestellt wird, bis der Gewindegrund - vorzugsweise in seiner Mitte - erreicht ist. Im Anschluß wird dieser Schlichtmeißel in der Z-Achse bei jedem Schnitt des Gewindeschneidzyklus um den Betrag der Spandicke so lange zugestellt, bis die entsprechende Flanke der Gewinderille fertig hergestellt ist. Dabei muss die schneidende Flanke des Drehmeißels in ihrer Winkellage derjenigen der Gewindezahnflanke entsprechen. Für die andere Seite der Gewinderille gilt entsprechendes für die dortige Gewindezahnflanke. Dieser Winkel ist bezüglich der schneidenden Kante des Drehmeißels ganz einfach über die Programmierung der B-Achse einstellbar. Dadurch kann für die beiden zu bearbeitenden Seiten derselbe Drehmeißel verwendet werden. Dies hat auch den Vorteil, dass eine aufwendige Synchronisation der Meißelspitze, wie sie bei zwei Werkzeugen erforderlich wäre, entfallen kann.

Da bei den CNC-Drehmaschinen gemäß dem Stand der Technik beim Gewindeschneiden eine automatische Kompensation des Schneidenradius nicht möglich ist, muss hier mit einem Trick gearbeitet werden, um die als Gewinderillengrund gebildete Kontur des Werkstückmantels ohne Verzerrung herstellen zu können. Dazu wird der Schneidenradiusmittelpunkt des jeweiligen Werkzeugs in der Werkzeugdatei beschrieben und eine um eine Äquidistante mit dem Abstand des Schneidenradius vergrößerte Kontur des Werkstücks programmiert.

Ähnlich muss vorgegangen werden, wenn im Programm der Maschine eine Funktion zur automatischen Korrektur der Werkzeugposition bei dynamisch schwenkender B-Achse - diese wird zum Teil ATC (automatical tool correction) genannt - nicht vorhanden ist. Ohne eine solche Korrektur würde sich wegen des wandernden B-Winkels die Position der Drehmeißelspitze ständig verändern und von der korrekten Position entfernen. Das Werkstück würde dann skurrile Formen annehmen und nicht mehr der Zielgeometrie entsprechen. Daher wird hiermit vorgeschlagen, in der Programmierung den Drehmittelpunkt der B-Achse des Kreuzschlittens der Maschine zu benutzen, und nicht wie üblich den Schneidenradiusmittelpunkt des Drehmeißels. Dazu wird der Abstand dieses Drehmittelpunktes vom jeweiligen Schneidenradiusmittelpunkt des Drehmeißels bestimmt und aus dieser Hypotenuse die Längen für die An- und Gegenkathete berechnet. Das so gebildete Korrekturdreieck muss nun noch um den Korrekturwinkel geschwenkt werden, welcher zwischen der schneidenden Flanke des Drehmeißels und der X-Achse des Kreuzschlittens eingeschlossen ist. Mit dem Schnittpunkt zwischen Gegenkathete und Hypotenuse wird dann die korrekte Position des Drehpunktes der B-Achse bestimmt.

Zum besseren Verständnis sollen im folgenden die der Erfindung gemäßen Einschraubkörper als auch das der Erfindung gemäße Verfahren anhand von schematischen Beispielen mit Hilfe von fünf Zeichnungsfiguren näher erläutert werden. Als Beispiel eines Einschraubkörpers mit gekrümmter Mantelfläche wurde dabei auf eine vereinfacht gezeichnete Hüftgelenkpfanne zurückgegriffen. Fig. 1 zeigt eine künstliche Hüftgelenkpfanne mit Gewinde gemäß dem Stand der Technik. In Fig. 2 ist eine solche Hüftgelenkpfanne mit einer konstanten Länge der polseitigen Zahnflanke dargestellt, während Fig. 3 eine vorteilhafte Ausführung zeigt, bei welcher die zwischen den Gewindezähnen liegenden Winkelhalbierenden eine konstante Höhe besitzen. In Fig. 4 ist die schematische Abwicklung zweier Gewindezähne eines Gewindezuges mit schwenkendem Teilflankenwinkel zu sehen, während die Fig. 5 die gleiche Situation mit einer überlagerten Rampen/Stufenfunktion verdeutlichen soll. Fig. 6 zeigt eine künstliche Hüftgelenkpfanne mit Gewinde gemäß dem Stand der Technik. In Fig. 7 ist eine erfindungsgemäße Hüftgelenkpfanne mit einem zum Pfannenpol gekippten Gewindezahnprofil und negativem Schrägungswinkel der Spannut dargestellt.

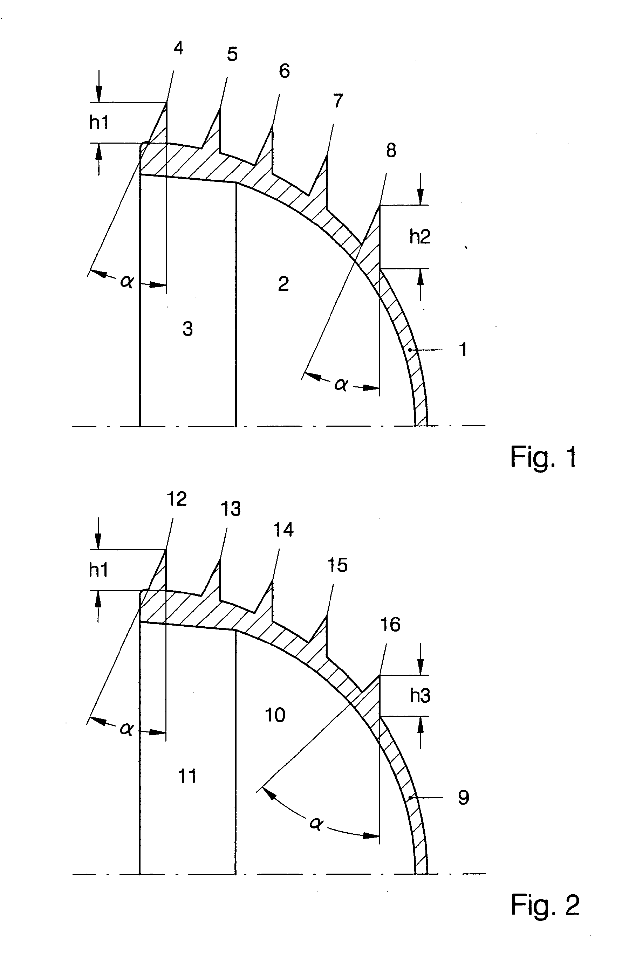

Fig. 1 soll das weiter oben beschriebene Phänomen verdeutlichen, welches bei gekrümmten Mantelflächen von Rotationskörpern mit einem gekippten Profil der Gewindezähne zu einer stärkeren Fluktuation der Gewindezahnhöhe führt. Zur Veranschaulichung wurde ein etwas vereinfachtes Ausführungsbeispiel einer künstlichen hemisphärischen Hüftgelenkpfanne mittlerer Größe mit einem konventionell hergestellten Schraubgewinde in einer geschnitten und halbseitig gezeichneten Darstellung in einem Maßstab von ungefähr 2,5:1 heran gezogen. Dabei wurde für die Gewindesteigung ein Wert von 5,2 mm gewählt. Die Gewindezähne wurden deutlich überhöht dargestellt, um die Details besser sichtbar machen zu können. Außerdem wurden sie der Einfachheit halber spitz zum Zahnkopf zulaufend und ohne Verrundung des Zahnfußes gezeichnet, obwohl in praktischen Ausführungen der Zahnkopf beschnitten und der Zahnfuß mit einem Übergangsradius versehen ist.

Die Hüftgelenkpfanne 1 ist aus einer Schale mit hemisphärischer äußerer Gestalt gebildet, welche in ihrer inneren Kontur in einen Bereich eines Kugelabschnitts 2 und eines Kegelabschnitts 3 aufgegliedert ist. Dieser innere Bereich ist für die Aufnahme eines Inletts vorgesehen. Die fünf Gewindezähne 4, 5, 6, 7, 8 besitzen ein unsymmetrisches Profil. Ihre zum Pol der Pfanne zeigenden Flanken weisen einen Winkel von 0°, und ihre zum Äquator der Pfanne zeigenden Flanken einen solchen von 24° auf. Entsprechend beträgt der eingeschlossene Flankenwinkel α 24°, wodurch sich ein Kippwinkel der Winkelhalbierenden von 12° in Richtung zum Pfannenpol ergibt. Diese Kipprichtung der Gewindezähne ist für die angestrebte Verwendung bei einer künstlichen Hüftgelenkpfanne besonders günstig, weil sich aufgrund der vorgegebenen Hauptbelastungsrichtung bessere Krafteinleitungsverhältnisse in das menschliche Becken erzielen lassen.

Bei dem gezeigten Beispiel folgt der Mantel der Pfannenschale mit hoher Genauigkeit der Kugelform. Diese ist allerdings nur im Bereich der Gewinderille präsent. Eine derartige Ausbildung erfordert spezielle Bearbeitungsverfahren, bei denen in der Regel Durchläufe mit zwei oder mehr Drehmeißeln mit unterschiedlichen Stirn- und/oder Seitenwinkeln und unterschiedlichen Bahnen bzw. relativen Verschiebungen der jeweiligen Drehmeißelposition erforderlich sind. Derartige Verfahren sind zum Beispiel aus der EP 0 480 551 B1 und WO 95/18586 A bekannt. Da dabei jedoch das Gewinde mit konstanter Steigung und konstanten Teilflankenwinkeln geschnitten wird, resultiert aus dem Kippwinkel der Gewindezähne eine fließende Veränderung der Gewindezahnhöhe. Es ist in der Fig. 1 gut zu erkennen, dass die radial gemessene Gewindezahnhöhe h2 in Polnähe fast doppelt so groß ist wie die Gewindezahnhöhe h1 in Äquatornähe. Obwohl fließende Zu- oder Abnahmen der Gewindezahnhöhen bis zu einem gewissen Grade besonders bei künstlichen Hüftgelenkpfannen durchaus gewollt sein können und dafür auch verschiedene Argumente ins Feld geführt werden, so ist doch eine solch starke Vergrößerung der Gewindezahnhöhe ausgesprochen nachteilig, weil damit unnötig tief in das Knochenmaterial eingegriffen wird. Derart große Gewindezähne an dem dem Pol nahen Gewindeanfang führen ferner insbesondere bei selbstschneidenden Schraubpfannen zu sehr hohen Einschraubkräften, da sich in dem Bereich des abrupten Anstiegs der Gewindezahnhöhe am Gewindebeginn die Zerspanungsarbeit beim Einschrauben in das Becken auf nur wenige Schneidkanten verteilt. Dadurch kann das Einschrauben der Hüftgelenkpfanne bis zum vollständigen Knochenkontakt in Frage gestellt sein.

Fig. 2 zeigt in einer mit Fig. 1 identischen Darstellungsweise das vereinfachte und leicht verzerrte Ausführungsbeispiel einer künstlichen Hüftgelenkpfanne mit Schraubgewinde gemäß der Erfindung. Die gezeigte Hüftgelenkpfanne 9 entspricht mit ihrer inneren Kontur aus Kugelabschnitt 10 und konischem Bereich 11 sowie der Zahl der Gewindezähne 12, 13, 14, 15, 16 jener aus Fig. 1. Der dem Äquator der Hüftgelenkpfanne nahe Gewindezahn 12 ist mit seiner Form und dem Betrag seiner Höhe h1 identisch mit dem entsprechenden Gewindezahn 4 aus Fig. 1. Der zwischen den Zähnen liegende Gewindegrund repräsentiert die Kugelform des Schalenmantels wie zuvor. Aufgrund der der Erfindung gemäßen Bearbeitung des Gewindes mittels zweier Herstellungsgänge, wobei die zum Äquator zeigende Flanke des Gewindezahns mit einem sich fließend ändernden Winkel geschnitten ist, wird nunmehr für den dem Pol nahen Gewindezahn 16 eine radial gemessene Zahnhöhe h3 verwirklicht, welche der Zahnhöhe h1 des dem Äquator nahen Zahns 12 entspricht. Dabei hat sich allerdings der eingeschlossene Flankenwinkel α des Gewindezahns von 24° auf etwa 46° vergrößert. Diese ausgesprochen derbe Vergrößerung des Flankenwinkels wurde hier allein deshalb gewählt, um das Prinzip der der Erfindung gemäßen Gestaltung und Bearbeitung besser verdeutlichen zu können.

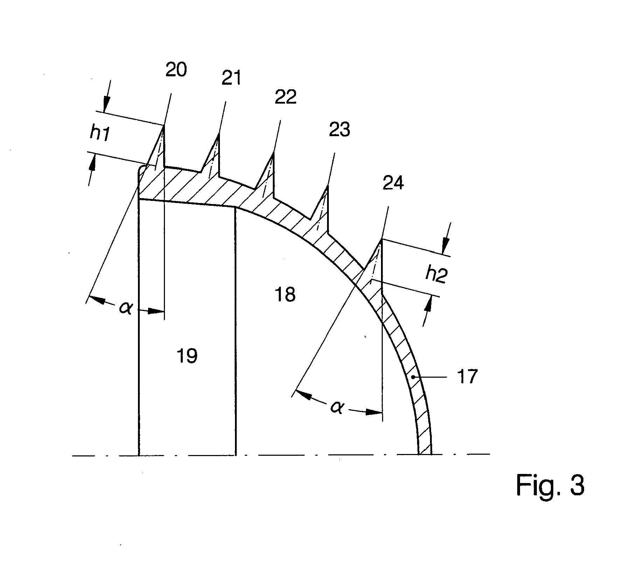

In der Fig. 3 ist die Variante einer künstlichen Hüftgelenkpfanne dargestellt, welche einer praktischen Ausführung deutlich näher kommt. Die Zeichnungsfigur zeigt in der schon aus den vorangehenden Figuren her bekannten Weise das Schnittbild einer Schraubpfanne 17 hemisphärischer Gestalt, deren Innenraum aus einem Kugelabschnitt 18 mit einem sich anschließenden konischen Bereich 19 gebildet ist. Die Schraubpfanne ist mit einem Gewinde ausgerüstet, von welchem sich im Schnittbild fünf Gewindezähne 20, 21, 22, 23, 24 ergeben. In das jeweilige Zahnprofil wurden die Winkelhalbierenden als strichpunktierte Linien eingezeichnet. Für das Zahnprofil des Gewindezahns 20 mit einem eingeschlossenen Flankenwinkel α von 24° beträgt der Kippwinkel dieser Winkelhalbierenden 12° in Richtung zum Pol. Bei dem Gewindezahn 24, welcher einen eingeschlossenen Flankenwinkel α von ca. 30° besitzt, hat sich der Kippwinkel der Winkelhalbierenden in Richtung zum Pol entsprechend auf etwa 15° vergrößert. In allen Fällen steht die zum Pfannenpol zeigende Flanke der Gewindezähne jeweils senkrecht auf der Pfannenachse. Die entlang der Winkelhalbierenden gemessenen Zahnhöhen, welche in der Zeichnungsfigur sowohl für den Gewindezahn 20 mit h1 als auch für den Gewindezahn 24 mit h2 eingezeichnet wurden, sind jeweils gleich groß.

In den Zeichnungsfiguren 2 und 3 wurden Ausführungsbeispiele gezeigt, bei denen mittels des der Erfindung gemäßen Verfahrens eine bestimmte Strecke des Zahnprofils auf eine konstante Länge gebracht worden ist. Das vorgeschlagene Verfahren ist darauf jedoch nicht beschränkt. Im Gegenteil eröffnet es durch eine bestimmte Festlegung auf eine springende und/oder gleitende Schwenkung der schneidenden Kante des Drehmeißels und einer entsprechenden Programmierung der B-Achse der CNC-Maschine zahlreiche Variationsmöglichkeiten, womit nicht nur exakt bestimmbare Zu- oder Abnahmen der Gewindezahnhöhen, sondern auch günstige Frei- und/oder Schneidwinkel an den Gewindezähnen verwirklicht werden können. Diese Modifikationen sind auch partiell in Teilbereichen des Gewindes einsetzbar.

Als Beispiel für eine derartige Modifikation des Gewindes sollen die Zeichnungsfiguren 4 und 5 herangezogen werden. Beide Zeichnungsfiguren sind sehr schematisch, im Maßstab vergrößert, und in Bezug auf den geometrischen Effekt stark überhöht dargestellt, um die Auswirkungen der speziellen Bearbeitung überhaupt erkennen zu können. Dabei werden vom jeweiligen Gewinde einer imaginären Hüftgelenkpfanne lediglich zwei aufeinander folgende Zähne gezeigt, und der Gewindegrund der Einfachheit halber weggelassen.

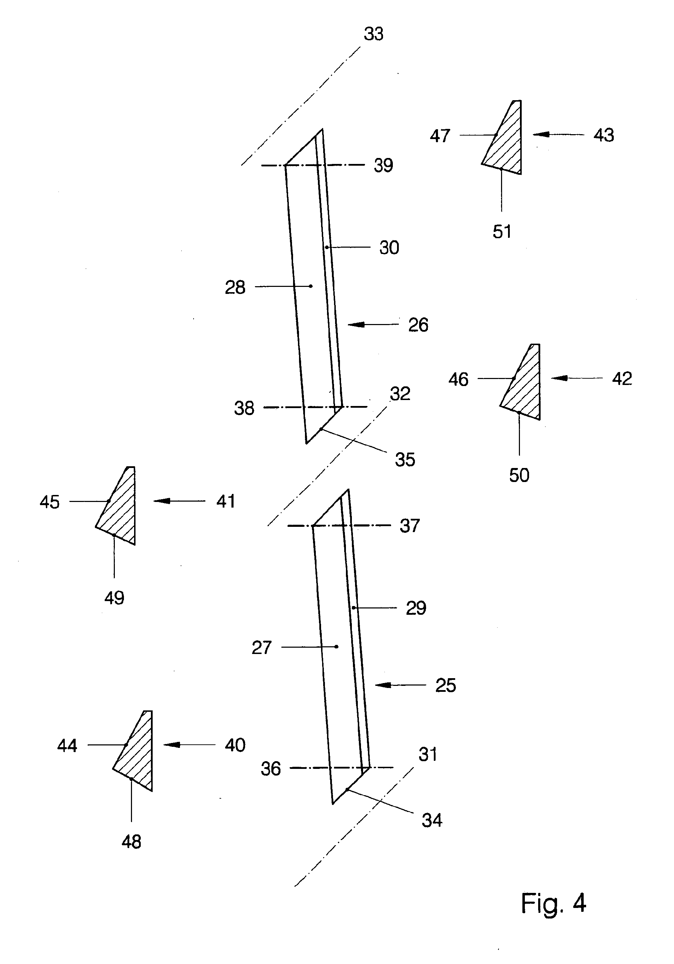

Fig. 4 zeigt demgemäß das Teilstück eines Gewindezuges mit einem Gewindezahn 25, welchem sich ein in Einschraubrichtung folgender Gewindezahn 26 anschließt. Beide Gewindezähne sind geometrisch auf den übertrieben gekrümmten Schalenmantel einer unsichtbaren Hüftgelenkpfanne aufgesetzt. Während die zum Pol zeigende Flanke beider Gewindezähne senkrecht auf dem Schalenmantel steht, weist die äquatorseitige Flanke 27, 28 eine jeweilige Neigung auf. Wegen der Beschneidung des Zahnkopfes besitzen beide Gewindezähne eine jeweilige Stirnfläche 29, 30. Die einzelnen Gewindezähne sind aus dem Gewindezug durch Ausarbeitung jeweiliger Spannuten entstanden, von denen die Mitten in der Zeichnung als strich-punktierte Linien 31, 32, 33 angedeutet sind. Wegen des schrägen Verlaufs der Spannuten sind an der jeweiligen Zahnfront Schneidkanten 34, 35 mit einem positiven Spanwinkel gebildet. Damit trotz einer konstanten Gewindesteigung eine gleich bleibende Höhe der Winkelhalbierenden des Gewindezahns erzielt wird, muß der zum Pfannenäquator zeigende Teilflankenwinkel gemäß der Erfindung entlang seiner Erstreckung mit einer Schwenkbewegung (z.B. der sogenannten B-Achse) überlagert werden. Dadurch wird ein sich kontinuierlich änderndes Gewindezahnprofil erzeugt. In der Zeichnungsfigur werden jeweils zwei Schnittbilder 40, 41 und 42, 43 der beiden Gewindezähne an den Schnittlinien 36, 37 und 38, 39 gezeigt. Wegen der gewählten extremen Krümmung des Schalenmantels ist die gebogene Zahnbasis 48, 49, 50, 51 in ihrer Neigung zum Pol der Hüftgelenkpfanne hin immer stärker gekippt. In der Zeichnungsfigur sind die einzelnen Neigungswinkel der zum Äquator zeigenden Gewindezahnflanken 44, 45, 46, 47 trotz der überhöhenden Darstellung mit 26,25°, 26,91°, 27,61° und 28,37° realisiert, und damit in ihrem jeweiligen Unterschied mit dem Auge kaum zu erkennen. Es versteht sich, dass bei real verwirklichten Gewinden der der Erfindung gemäßen Art trotz der kleinen Winkeländerungsinkremente in der Addition deutliche Größenordnungen erreicht werden. Typische Teilflankenverläufe zeigen z.B. Teilflankenänderungen in Höhe von etwa 9° über den Gewindebereich.

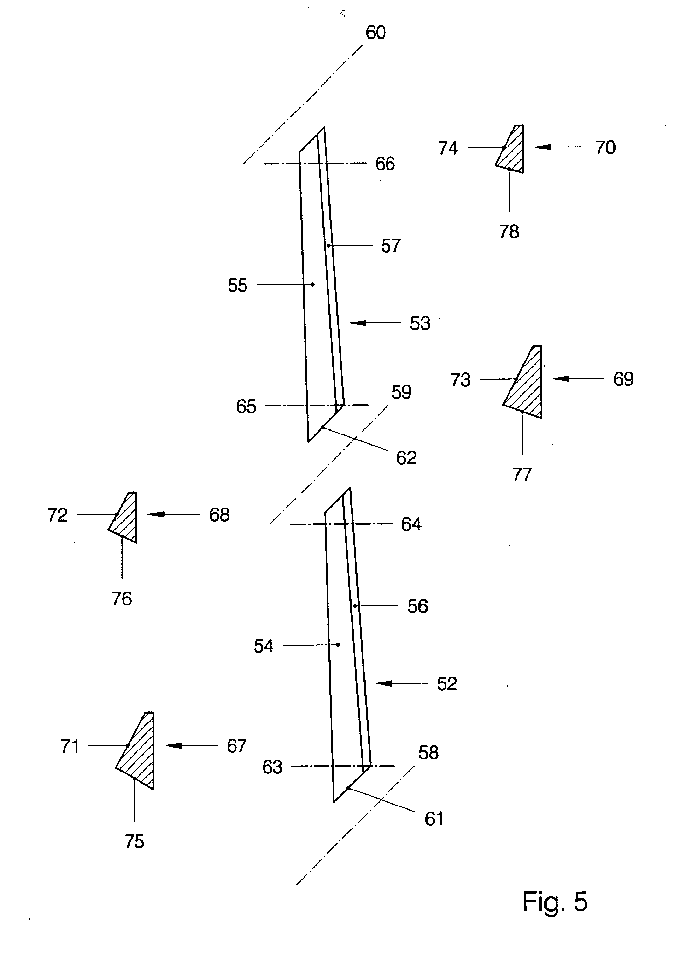

Die oben beschriebene Gewindegestaltung ist für sich allein nicht zufriedenstellend, weil während des Einschraubvorgangs wegen der fehlenden Freiwinkel an den Gewindezähnen mit Klemmeffekten zu rechnen ist und außerdem die jeweils gegenüber der Gewindezahnflanke nicht hervortretende Schneidkante keine Wirkung entfalten kann. Zur Beseitigung dieses Problems wird mit weiterer Erfindung vorgeschlagen, dem Verlauf des Gewindezuges eine mit der Zahnfolge synchronisierte Funktion in Gestalt von Rampen und Stufen, oder dergleichen so zu überlagern, dass hinter der jeweiligen an der Zahnfront gebildeten Schneidkante wahlweise ein Neutral- oder Freiwinkel und gleichzeitig eine exponierte Position der Schneidkante gebildet ist. Diese Modifikation wird in Fig. 5 gezeigt.

Fig. 5 lehnt sich stark an die Fig. 4 an, wobei auch hier die gezeigten Abmessungen und geometrischen Effekte zum Zwecke des besseren Verständnisses deutlich überhöht dargestellt wurden. Der Fig. 4 gleichend werden zwei Gewindezähne 52, 53 mit schrägen Flanken 54, 55 und Stirnflächen 56, 57 gezeigt. Wie zuvor sind diese Gewindezähne durch das Einbringen von Spannuten, hier angedeutet durch strich-punktierte Mittellinien 58, 59, 60, voneinander getrennt. Es sind wiederum Schnittlinien 63, 64, 65, 66 eingezeichnet und die entsprechenden Schnitte 67, 68, 69, 70 dargestellt. Die jeweilige Zahnbasis 75, 76, 77, 78 ist in ihrer Winkellage - so weit diese noch existent ist - mit derjenigen aus Fig. 4 identisch. Auch die zum Äquator zeigenden Flanken 71, 72, 73, 74 wurden mit ihrem Teilflankenwinkel und dessen schwenkendem Verlauf beibehalten. Allerdings wurde diesem Verlauf eine Treppenfunktion derart überlagert, dass das Gewindezahnprofil nun hinter seiner Zahnfront mit der jeweiligen Schneidkante 61, 62 einer stetigen Verschmälerung und Höhenabnahme relativ zum Schalenmantel der Hüftgelenkpfanne unterliegt. Der seitliche Versprung zwischen den einzelnen zum Äquator zeigenden Flanken der Gewindezähne sowie der höhenmäßige Versprung des jeweiligen Zahnkopfes findet in Bezug auf die spanende Herstellung und damit auch am fertigen Produkt im Bereich der jeweiligen Spannut statt und wird in der Zeichnungsfigur durch die Exposition der jeweiligen Schneidkante gegenüber dem vorauslaufenden Gewindezahn deutlich. Erst dadurch wird ein effektiver Schnitt der Schneidkanten realisiert und ein Verklemmen der Gewindezähne während des Einschraubens sicher unterbunden.

Der erfindungsgemäße Einschraubkörper der sich dadurch auszeichnet, dass die in Einschraubrichtung liegende Flanke des Gewindezahns einen konstanten Winkel besitzt und die andere Flanke des Gewindezahns im genannten Teilbereich einer Winkeländerung unterliegt, ist insbesondere in Kombination mit einem linksgedrallten Gewinde günstig. Bei konstanter Gewindesteigung wird bei Kippen des Zahns zum Pol die äquatorseitige Flanke zur schiebenden Kante. Auf der äquatorseitigen Flanke ist dann auch günstigerweise die Schneidkante ausgebildet. Dadurch ergibt ein linksgedralltes Gewinde.

Die oben beschriebene, dem Gewindezug überlagerte Sprungfunktion aus Rampe und Stufe ist mittels der üblichen Drehverfahren nicht herstellbar. Daher wird hiermit vorgeschlagen, die spanende Bearbeitung in wenigstens zwei Schritte aufzugliedern. Dabei wird zuerst mittels CNC-Drehen die fließende Kontur der entsprechenden Gewindezahnflanke hergestellt, z.B. so wie in Fig. 4 zu sehen. Dann schließt sich für die Erzeugung der jeweils überlagerten aus Rampe und Stufe gebildeten Funktion ein Fräsgang an, welcher nur relativ wenig Material abtragen muß. Im Prinzip kann diese Bearbeitung auch mittels des vom Anmelder so genannten Humpel-Drehverfahrens ausgeführt werden, wie es in der EP 1 051 131 A1 und EP 1 318 803 A1 beschrieben ist.

Bezüglich der praktischen Umsetzung der Erfindung besteht eine sehr große Bandbreite hinsichtlich des Zahnprofils (Zahnhöhe, eingeschlossener Flankenwinkel, Kippwinkel, Form und Winkel des Zahnkopfes) und dessen Verlaufs (Gewindesteigung, relative Höhenabnahme, Änderung des Teilflankenwinkels). Dadurch wird mit der Erfindung ein sehr flexibel einsetzbares Mittel für die optimale Anpassbarkeit von Spezialgewinden, insbesondere von in ihrer Erstreckung gekrümmten oder geknickten Spezialgewinden, zur Verfügung gestellt.

Die Fig. 6 zeigt die schematische Darstellung einer künstlichen Hüftgelenkpfanne 79 gemäß dem Stand der Technik in einem leicht vergrößerten Maßstab. Das gezeigte Beispiel besitzt eine sphärische Kontur des gekrümmten Schalenmantels. Es sind vier umlaufende Zahnrippen 81, 84, 83 und 84 zu sehen, welche durch eine einzige Spannut 86 unterbrochen sind. Die anderen Spannuten wurden aus Vereinfachungsgründen weggelassen. Die Mittelachse der Hüftgelenkpfanne ist durch eine strichpunktierte Linie 80 und die Mitte der Spannut durch eine strichpunktierte Linie 85 angedeutet. Zwischen beiden ist der negative Schrägungswinkel α gebildet. Der Schrägungswinkel entspricht seiner winkelmäßigen Größe nach dem Steigungswinkel des Gewindes, so daß die an den Gewindezähnen durch die Spannut gebildeten Schneidkanten in Bezug auf die Vorschubrichtung beim Einschrauben quer zur Erstreckung der Gewinderippen, und damit neutral stehen.

In der Fig. 7 ist eine der Erfindung gemäße künstliche Hüftgelenkpfanne 87 gleicher Größe und Form dargestellt. Wie zuvor wurde aus Vereinfachungsgründen lediglich eine einzige Spannut 94 eingezeichnet. Auch auf die Darstellung eines Dralls wurde wegen des zeichnerischen Aufwands verzichtet. Das aus vier Umläufen 89, 90, 91 und 92 bestehende Gewinde besitzt ein unsymmetrisches Zahnprofil, welches zum Pfannenpol hin geneigt ist. Diese Kipprichtung bewirkt - wie weiter oben erläutert - eine Vergleichmäßigung der zwischen Implantat und knöchernem Lager zu über-tragenden Kräfte. Die Spannut ist in ihrem Winkel demjenigen der Gewindesteigung entgegengerichtet. Mit ihrer strichpunktierten Mittellinie 93 schließt sie gegen die Achse 88 der Hüftgelenkpfanne einen Winkel α von 45° ein. Die an den Gewinderippen gebildeten Schneidkanten liegen nun auf der Rückseite der Gewinderippen auf den zum Pfannenäquator gerichteten Gewindezahnflanken und besitzen wegen den stark schräg verlaufenden Spannuten jeweils einen stark positiven Spanwinkel. Durch die gegenüber dem Stand der Technik günstigere Platzierung der Schneidkanten kommen diese in Verbindung mit dem positiven Spanwinkel voll zur Geltung und bewirken so eine deutliche Senkung des Einschraubdrehmoments und gleichzeitig eine Steigerung der Überdrehreserve.

## Patentansprüche

1. Einschraubkörper mit einer mindestens in einem Teilbereich nicht-linearer Mantelfläche und einem mindestens in diesem Teilbereich liegenden Gewinde mit sich änderndem, aus Gewindezahn (12-16, 20-24, 25, 26) und Gewinderille gebildeten Gewindeprofil, wobei die in Einschraubrichtung liegende Flanke des Gewindezahns zu einer Senkrechten zur Mittelachse (80, 88) des Einschraubkörpers einen konstanten Winkel besitzt,
**dadurch gekennzeichnet,**
**dass** die andere Flanke (27, 28, 44-47) des Gewindezahns im genannten Teilbereich zu der genannten Senkrechten zur Mittelachse (80, 88) des Einschraubkörpers einer Winkeländerung unterliegt.

2. Einschraubkörper gemäß Anspruch 1, **dadurch gekennzeichnet, dass** die in Einschraubrichtung liegende Flanke des Gewindezahns (12-16, 20-24, 25, 26) subvertikal, vorzugsweise senkrecht, zur Mittelachse (80, 88) des Einschraubkörpers steht.

3. Einschraubkörper gemäß einem der Ansprüche 1 oder 2, **dadurch gekennzeichnet, dass** die Winkeländerung der anderen Flanke (27, 28, 44-47) zu der genannten Senkrechten zur Mittelachse (80, 88) des Einschraubkörpers kontinuierlich erfolgt.

4. Einschraubkörper gemäß einem der Ansprüche 1 bis 3, **dadurch gekennzeichnet, dass** dem Verlauf der Gewindezähne (52, 53) eine jeweils aus mindestens einer Rampe und einer Stufe gebildete geometrische Funktion überlagert ist.

5. Einschraubkörper gemäß Anspruch 4, **dadurch gekennzeichnet, dass** die Höhe des Gewindezahnprofils relativ zur Mantelfläche des Einschraubkörpers im hinter einer jeweiligen Schneidkante (61, 62) liegenden Bereich abnimmt.

6. Einschraubkörper gemäß einem der vorhergehenden Ansprüche, **dadurch gekennzeichnet, dass** die Schneidkante (35) jedes Zahns (26) im Vergleich zur Schneidkante (34) des vorhergehenden Zahns (25) äquatorseitig hervorsteht.

7. Einschraubkörper gemäß einem der Ansprüche 1 bis 6, **dadurch gekennzeichnet, dass** der Einschraubkörper als Implantat realisiert ist.

8. Einschraubkörper gemäß Anspruch 7, **dadurch gekennzeichnet, dass** der Einschraubkörper als Hüftgelenkpfanne (9, 17) realisiert ist.

9. Verfahren zur spanenden Herstellung von Gewinden mit sich mindestens in einem Teilbereich verändernden Gewindeprofil auf einer CNC-Maschine, vorzugsweise einer CNC-Drehmaschine, wobei das Gewinde mindestens in diesem Teilbereich mit mindestens zwei Herstellungsgängen bzw. -zyklen bearbeitet wird,
**dadurch gekennzeichnet,**
**dass** bei einem dieser Herstellungsgänge bzw. -zyklen die die Gewindezahnflanke schneidende Kante des Drehmeißels zu einer Senkrechten zur Mittelachse (80, 88) des Einschraubkörpers einen festen Winkel einnimmt, und bei dem anderen Herstellungsgang bzw. -zyklus die die andere Gewindezahnflanke schneidende Kante des Drehmeißels zu der genannten Senkrechten zur Mittelachse (80, 88) des Einschraubkörpers in ihrem Winkel während des Durchlaufs durch die Gewinderille geändert wird.

10. Verfahren gemäß Anspruch 9, **dadurch gekennzeichnet, dass** der eine Bearbeitungsgang bzw. -zyklus im wesentlichen die eine Flanke des Gewindezahns sowie einen Teil des benachbarten Gewindegrundes, und der andere Bearbeitungsgang bzw. -zyklus im wesentlichen die andere Flanke des Gewindezahns sowie einen Teil des dieser anderen Flanke benachbarten Gewindegrundes betrifft.

11. Verfahren gemäß Anspruch 9 oder 10, **dadurch gekennzeichnet, dass** das Gewinde mit drei verschiedenen Bearbeitungsgängen bzw. -zyklen bearbeitet wird, wovon mit dem dritten Bearbeitungsgang bzw. -zyklus im wesentlichen die Mitte der Gewinderille bearbeitet und dabei eine Winkeleinstellung für den Drehmeißel benutzt wird, welche zwischen den für die beiden anderen Bearbeitungsgänge bzw. -zyklen benutzten Winkeleinstellungen liegt.

12. Verfahren gemäß Anspruch 11, **dadurch gekennzeichnet, dass** der dritte Bearbeitungsgang zuerst ausgeführt wird.

13. Verfahren gemäß einem der vorgenannten Ansprüche 9 bis 12, **dadurch gekennzeichnet, dass** die Winkeländerung des die andere Gewindezahnflanke schneidenden Drehmeißels während des Durchlaufs durch die Gewinderille kontinuierlich erfolgt.

14. Verfahren gemäß einem der Ansprüche 9 bis 13, **dadurch gekennzeichnet, dass** die Winkeländerung des Drehmeißels mittels Drehung der B-Achse der CNC-Maschine, vorzugsweise einer CNC-Drehmaschine, realisiert ist.

15. Verfahren gemäß Anspruch 9 bis 14, **dadurch gekennzeichnet, dass** zuerst die vom Schneidenradiusmittelpunkt des Drehmeißels während des Abfahrens der in Gestalt des Gewinderillengrundes zu realisierenden Mantelfläche des herzustellenden Einschraubkörpers zu beschreibende Bahn berechnet wird, diese Bahn mit einem jeweiligen Offset für X und Z in Bezug auf den Abstand des Schneidenradiusmittelpunktes des Drehmeißels vom Rotationsmittelpunkt der B-Achse des Kreuzschlittens und für B in Bezug auf die Winkeldifferenz zwischen der Schneide des Drehmeißels und der Nullstellung der B-Achse umgerechnet und im CNC-Programm angewendet wird, und in der Werkstückbeschreibung eine Äquidistante verwendet ist, welche in ihrem Betrag dem Schneidenradius des Drehmeißels entspricht.

16. Verfahren gemäß einem oder mehreren der vorstehenden Ansprüche 9 bis 15, **dadurch gekennzeichnet, dass** der Relativbewegung zwischen Werkstück und Werkzeug eine mit der Zahnfolge synchronisierte Rampen- und Stufenfunktion überlagert ist.

17. Verfahren gemäß Anspruch 16, **dadurch gekennzeichnet, dass** die Zerspanung frästechnisch erfolgt.

## Claims

1. A screw-in element having a lateral surface that is nonlinear in at least one partial area and a thread lying at least in this partial area having a changing thread profile formed by thread tooth (12-16, 20-24, 25, 26) and thread groove, wherein the flank of the thread tooth lying in the screwing direction has a constant angle to a vertical to the center axis (80, 88) of the screw-in element,
**characterized in that**
the other flank (27, 28, 44-47) of the thread tooth in said partial area is subject to a change in the angle to said vertical to the center axis (80, 88) of the screw-in element.

2. The screw-in element according to claim 1, **characterized in that** the thread tooth's (12-16, 20-24, 25, 26) flank lying in the screwing direction is subvertical, preferably perpendicular, to the center axis (80, 88) of the screw-in element.

3. The screw-in element according to claim 1 or 2, **characterized in that** the other flank's (27, 28, 44-47) change in angle to the cited vertical to the center axis (80, 88) of the screw-in element occurs continuously.

4. The screw-in element according to any one of claims 1 to 3, **characterized in that** the curve of the thread teeth (52, 53) is superimposed by a geometrical function formed in each case from at least one ramp and one step.

5. The screw-in element according to claim 4, **characterized in that** the height of the thread tooth profile relative to the lateral surface of the screw-in element decreases in the area lying behind a respective cutting edge (61, 62).

6. The screw-in element according to any one of the preceding claims, **characterized in that** the cutting edge (35) of each tooth (26) projects on the equator side in comparison to the cutting edge (34) of the preceding tooth (25).

7. The screw-in element according to any one of claims 1 to 6, **characterized in that** the screw-in element is implemented as an implant.

8. The screw-in element according to claim 7, **characterized in that** the screw-in element is implemented as a hip joint socket (9, 17).

9. A method for machining threads which have a thread profile changing at least in a partial area, on a CNC machine, preferably a CNC lathe, wherein the thread is machined at least in this partial area in at least two production steps and/or cycles,
**characterized in that**
in one of these production steps and/or cycles, the edge of the lathe chisel cutting the thread tooth flank assumes a fixed angle to a vertical to the center axis (80, 88) of the screw-in element, and in the other production step and/or cycle, the edge of the lathe chisel cutting the other thread tooth flank is changed in its angle to said vertical to the center axis (80, 88) of the screw-in element during the passage through the thread groove.

10. The method according to claim 9, **characterized in that** the one machining step and/or cycle substantially relates to the one flank of the thread tooth and a part of the adjacent thread base, and the other machining step and/or cycle substantially relates to the other flank of the thread tooth and a part of the thread base adjacent to this other flank.

11. The method according to claim 9 or 10, **characterized in that** the thread is machined in three different machining steps and/or cycles, wherein the third machining step and/or cycle is used to machine substantially the middle of the thread groove and an angle setting is used for the lathe chisel which lies between the angle settings used for the two other machining steps and/or cycles.

12. The method according to claim 11, **characterized in that** the third machining step is carried out first.

13. The method according to any one of the preceding claims 9 to 12, **characterized in that** the angle change of the lathe chisel cutting the other thread tooth flank is performed continuously during the passage through the thread groove.

14. The method according to any one of claims 9 to 13, **characterized in that** the angle change of the lathe chisel is implemented by means of the rotation of the B axis of the CNC machine, preferably a CNC lathe.

15. The method according to claims 9 to 14, **characterized in that** first the path to be followed by the cutting radius center point of the lathe chisel during the travel along the lateral surface, to be implemented in the form of the thread groove base, of the screw-in element to be produced is calculated, this path is converted using a particular offset for X and Z in relation to the distance of the blade radius center point of the lathe chisel from the rotation center point of the B axis of the compound slide and for B in relation to the angular difference between the blade of the lathe chisel and the zero setting of the B axis and is applied in the CNC program, and a cutter center line is used in the workpiece description whose absolute value corresponds to the blade radius of the lathe chisel.

16. The method according to one or more of the preceding claims 9 to 15, **characterized in that** the relative movement between workpiece and tool is superimposed by a ramp and step function that is synchronized with the tooth sequence.

17. The method according to claim 16, **characterized in that** machining is performed by milling.

## Revendications

1. Corps à visser comprenant une surface d'enveloppe au moins non-linéaire dans une zone partielle et un filet reposant au moins dans cette zone partielle présentant un profil fileté variable formé de dentés filetées (12-16, 20-24, 25, 26) et de rainures filetées, sachant que le flanc de la dent filetée placé dans le sens de vissage possède un angle constant par rapport à une perpendiculaire à l'axe central (80, 88) du corps à visser,
**caractérisé en ce que**
l'autre flanc (27, 28, 44-47) de la dent filetée subit une modification d'angle dans ladite zone partielle par rapport à ladite perpendiculaire à l'axe central (80, 88) du corps à visser.

2. Corps à visser selon la revendication 1, **caractérisé en ce que** le flanc de la dent filetée (12-16, 20-24, 25, 26) placé dans le sens de vissage est sous-vertical, de préférence perpendiculaire à l'axe central (80, 88) du corps à visser.

3. Corps à visser selon la revendication 1 ou 2, **caractérisé en ce que** la modification d'angle de l'autre flanc (27, 28, 44-47) par rapport à ladite perpendiculaire à l'axe central (80, 88) du corps à visser est continue.

4. Corps à visser selon l'une des revendications 1 à 3, **caractérisé en ce qu'**une fonction géométrique formée respectivement d'au moins une rampe et d'un étage est superposée au tracé des dents filetées (52, 53).

5. Corps à visser selon la revendication 4, **caractérisé en ce que** la hauteur du profil de dent filetée par rapport à la surface d'enveloppe du corps à visser décroît dans la zone à l'arrière d'une arête de coupe respective (61, 62).

6. Corps à visser selon l'une des revendications précédentes, **caractérisé en ce que** l'arête de coupe (35) de chaque dent (26) fait saillie côté équateur par rapport à l'arête de coupe (34) de la dent (25) précédente.

7. Corps à visser selon l'une des revendications 1 à 6, **caractérisé en ce que** le corps à visser est fabriqué en tant qu'implant.

8. Corps à visser selon la revendication 7, **caractérisé en ce que** le corps à visser est fabriqué en tant que cavité cotyloïde (9, 17).

9. Procédé destiné à la fabrication par copeaux de filets présentant un profil fileté variable au moins dans une zone partielle, sur une machine à commande numérique, de préférence sur un tour à commande numérique, dans lequel le filet au moins dans une zone partielle est usiné avec au moins deux passes, respectivement, cycles de fabrication,
**caractérisé en ce que**
lors de ces passes, respectivement, cycles de fabrication, l'arête de l'outil de tour coupant le flanc de la dent filetée, par rapport à une perpendiculaire à l'axe central (80, 88) du corps à visser, adopte un angle fixe, et dans l'autre passe, respectivement, cycle de fabrication, l'arête de l'outil de tour coupant l'autre flanc de la dent filetée par rapport à la perpendiculaire à l'axe central (80, 88) du corps à visser est modifiée dans son angle par la rainure filetée lors de l'exécution.

10. Procédé selon la revendication 9, **caractérisé en ce que** l'une passe, respectivement, l'un cycle d'usinage concerne essentiellement l'un flanc de la dent filetée ainsi qu'une partie du fond de filet voisin et l'autre passe, respectivement, cycle d'usinage concerne essentiellement l'autre flanc de la dent filetée ainsi qu'une partie du fond de filet voisin de cet autre flanc.

11. Procédé selon la revendication 9 ou 10, **caractérisé en ce que** le filet est usiné avec trois passes, respectivement cycles d'usinage différent(e)s, le centre de la rainure filetée étant essentiellement usiné par la troisième passe, respectivement, le troisième cycle d'usinage, et un réglage d'angle de l'outil de tour est alors utilisé qui est situé entre les réglages d'angles utilisés pour les deux autres passes, respectivement, cycles d'usinage.

12. Procédé selon la revendication 11, **caractérisé en ce que** le troisième cycle d'usinage est exécuté en premier.

13. Procédé selon l'une des revendications précédentes 9 à 12, **caractérisé en ce que** la modification d'angle de l'outil de tour coupant l'autre flanc de dent filetée a lieu en continu pendant l'exécution par la rainure filetée.

14. Procédé selon l'une des revendications 9 à 13, **caractérisé en ce que** la modification d'angle de l'outil de tour est effectuée par une rotation de l'axe B de la machine à commande numérique, de préférence d'un tour à commande numérique.

15. Procédé selon l'une des revendications 9 à 14, **caractérisé en ce que** la trajectoire à décrire par le point médian du rayon de coupe de l'outil de tour pendant l'enlèvement de la surface d'enveloppe du corps à visser à réaliser à la forme du fond de rainure filetée est d'abord calculée, cette trajectoire est convertie avec un décalage respectif pour X et Z par rapport à la distance du point médian du rayon de coupe de l'outil de tour au point médian de rotation de l'axe B du chariot porte-outil à mouvements croisés, et pour B par rapport à la différence d'angle entre la coupe de l'outil de tour et le point zéro de l'axe B, et appliquée dans le programme de commande numérique, et une équidistante est employée dans la description de la pièce, laquelle correspond dans sa valeur absolue au rayon de coupe de l'outil de tour.

16. Procédé selon l'une ou plusieurs des revendications précédentes 9 à 15, **caractérisé en ce qu'**une fonction de rampe et d'étage synchronisée est superposée au mouvement relatif entre la pièce et l'outil.

17. Procédé selon la revendication 16, **caractérisé en ce que** l'usinage par enlèvement de copeaux est effectué par fraisage.
